# EUROPÄISCHE PATENTSCHRIFT

(11) **EP 1 424 982 B1**
(45) Veröffentlichungstag und Bekanntmachung des Hinweises auf die Patenterteilung: **19.01.2005**
(21) Anmeldenummer: 02762395.8
(22) Anmeldetag: 25.07.2002
(51) Int. Cl.: A61F 13/537, A61F 13/539, A61F 13/536

(54) **SAUGKÖRPER FÜR HYGIENEARTIKEL**
ABSORBENT BODY FOR HYGIENE ARTICLES
CORPS ABSORBANT POUR ARTICLES HYGIENIQUES

(30) Priorität: 08.09.2001 DE 10144128
(43) Veröffentlichungstag der Anmeldung: 09.06.2004
(73) Patentinhaber: Paul Hartmann Aktiengesellschaft, 89522 Heidenheim (DE)
(72) Erfinder: MANGOLD, Rainer, 89542 Herbrechtingen (DE)
(74) Vertreter: Friz, Oliver
(86) Internationale Anmeldenummer: PCT/EP2002/008284
(87) Internationale Veröffentlichungsnummer: WO 2003/022194

(56) Entgegenhaltungen:
- EP-A- 0 291 316
- EP-A- 0 810 078
- WO-A-97/13484
- US-A- 4 590 114
- US-A- 4 605 402
- US-A- 5 360 420

## Beschreibung

Die Erfindung betrifft einen wenigstens zweischichtigen Saugkörper für Hygieneartikel, insbesondere für Damenbinden oder Slipeinlagen, mit einer körperzugewandten Flüssigkeitsaufnahme- und -verteilerschicht aus einem ersten Fasermaterial mit einer ersten Porengröße und einer körperabgewandten Flüssigkeitsspeicherschicht mit wenigstens 20 bis 98 Gew.-% Zellstofffasern mit einer zweiten Porengröße, die gegenüber der ersten geringer ist.

Ein derartiger Saugkörperaufbau ist aus einer Vielzahl von Druckschriften, beispielsweise EP 0 512 010 B1 bekannt. Bei diesem bekannten Saugkörper ist die körperzugewandte Flüssigkeitsaufnahme- und -verteilerschicht aus intravernetzten Zellstofffasern, sogenanntes Curled-Fiber-Fasermaterial gebildet, welches gerade im eingenässten Zustand ein großes Porenvolumen behält und daher zur raschen Aufnahme großer Flüssigkeitsmengen dient, ohne dass kapillare Zwischenräume blockiert werden und die auftreffende Flüssigkeit zu den Seiten hin wegläuft. Bei diesem Curled-Fiber-Fasermaterial sind die Fasern durch innerhalb einer Zellulosefaser ausgebildeten Wasserstoffbrückenbindungen in sich versteift und das Fasermaterial tendiert bei einer geeigneten Vorverdichtung sogar dazu, im eingenässten Zustand aufzugehen, also noch voluminöser zu werden. Andere Fasermaterialien, die sich zur Bildung der körperzugewandten Flüssigkeitsaufnahme- und -verteilerschicht eignen, sind beispielsweise thermisch verfestigte thermoplastische Bikomponentenfasern, die infolge der thermischen Verfestigung bei Flüssigkeitsbeaufschlagung nicht zum Zusammenfallen neigen.

Aufgabe der körperzugewandten Flüssigkeitaufnahme- und - verteilerschicht ist es, auftreffende Flüssigkeit aufzunehmen, zwischenzuspeichern und innerhalb der Schicht zu verteilen und dann an die darunter liegende demgegenüber mit einer geringeren Porengröße und damit einer höheren Kapillarität ausgebildeten Speicherschicht abzugeben. Je größer die Porengröße einer Faserschicht ist, desto geringer ist das Vermögen, Flüssigkeit innerhalb der Schicht aufgrund kapillarer Mechanismen zu transportieren, d. h., desto geringer ist die Kapillarität dieser Schicht und umgekehrt. Durch die geringen kapillaren Faserzwischenräume in der Speicherschicht wird die Flüssigkeit dann infolge kapillaren Flüssigkeitstransports innerhalb der Speicherschicht zu noch ungebrauchten Bereichen des Saugkörpers transportiert. Dieser Transportvorgang benötigt jedoch eine gewisse Zeit, weshalb schnell absorbierende Flüssigkeitsaufnahme- und - verteilerschichten der vorstehend genannten Art verwendet werden.

Die verwendeten Schichten sollten in geeigneter Weise miteinander verbunden werden, um die Handhabung von flächenhaftem Bahnmaterial zu vereinfachen, aber auch um bei der Benutzung eines Hygieneartikels das Verrutschen der Schichten gegeneinander zu verhindern. Hierzu sind eine Vielzahl von Verfahren und Mittel bekannt, wie z. B. die Verwendung von Haftklebern, thermische Verfestigung von Faserbahnen unter Hinzufügung thermoplastischen Fasermaterials oder von Klebematerialien oder die Anbringung von Schweissverbindungen. Ferner ist die Verbindung von flächenhaftem Bahnmaterial durch Kalandrieren als dem Fachmann bekannte Maßnahme jedenfalls grundsätzlich bekannt, wobei hierbei eine möglicherweise ungewollte Verdichtung des Bahnmaterials entsteht.

Aus EP 1 032 342 B1 ist es beispielsweise bekannt, zur bindestofffreien Herstellung einer aus Zellulosefasern bestehenden Faserstoffbahn ausgehend von einem luftgelegten Zellulosefaservlies dieses Vlies durch den Spalt eines Kalanderrollenpaares hindurchzuführen, wobei das Kalanderrollenpaar punktförmige, gegeneinander nahezu anlegbare Erhebungen aufweist, die zur Herstellung von beidseitigen Prägepunkten in dem Vlies und damit zur Verfestigung des Vlieses führen.

Der vorliegenden Erfindung liegt die Aufgabe zugrunde, bei einem zweischichtigen Saugkörper der eingangs genannten Art eine zufriedenstellende Verbindung der Schichten zu erreichen, wobei die Verschiedenheit der Porengrößen der körperzugewandten Aufnahme- und Verteilerschicht und der körperabgewandten Speicherschicht weitestgehend beibehalten werden sollen, so dass sich ein gutes Flüssigkeitsaufnahme-, -verteiler- und -speicherverhalten des Saugkörpers erreichen lässt.

Diese Aufgabe wird durch einen Saugkörper der eingangs genannten Art gelöst, wobei das erste Fasermaterial der körperzugewandten Flüssigkeitsaufnahme- und -verteilerschicht aus luftgelegten intravernetzten Zellulosefasern besteht und wobei die Schichten durch eine Vielzahl von rasterartig vorgesehenen Prägepunkten klebstofffrei miteinander verbunden sind, und wobei dadurch in der Flüssigkeitsaufnahme- und - verteilerschicht um die Prägepunkte herum ein trichterförmiger Porengradient in dieser Schicht ausgebildet ist, der die Flüssigkeitseinleitung in die darunter liegende Speicherschicht unterstützt.

Mit der Erfindung wurde festgestellt, dass sich durch das Verbinden der wenigstens zwei Schichten durch ein punktförmiges Prägemuster in der großporigen Flüssigkeitsaufnahme- und -verteilerschicht eine hervorragende Flüssigkeitshandhabungscharakteristik erreichen lässt. Durch das punktförmige Verprägen der Schichten wird im Bereich zwischen den Prägepunkten ein großporiger Faserzwischenraum, also ein großes Flüssigkeitsaufnahmevolumen, beibehalten. Durch die Prägepunkte wird um diese herum ein im wesentlicher trichterförmiger Porengradient, also eine zunehmende Verdichtung mit entsprechend einhergehender abnehmender Porengröße in einem trichterförmigen Bereich um den jeweiligen Prägepunkt herum in Richtung auf das Prägezentrum ausgebildet, welches jeweils als Sog- oder Drainagestelle innerhalb der großporigen Flüssigkeitsaufnahmeschicht wirkt und zur punktuellen Entwässerung und Flüssigkeitseinleitung in die darunter liegende Speicherschicht führt. Wenn vorstehend von einem trichterförmigen Porengradienten und einem trichterförmigen Bereich die Rede ist, so soll hierdurch zum Ausdruck gebracht werden, dass dieser Bereich um einen jeweiligen Prägepunkt herumgehend zu verstehen ist; er muss nicht notwendigerweise streng trichter- oder konusförmig sein, sondern er folgt der auf den jeweiligen Prägepunkt hin gerichteten Geometrie der Prägestelle, die jedoch gleichwohl im Sinne eines Trichters auf ihr Zentrum hin gerichtet ist.

Die rasterartige Vorsehung der Prägepunkte führt also nicht nur zur Verbindung der wenigstens zwei Schichten, also zur Herstellung eines vorzugsweise bindemittelfreien Schichtenverbunds, sondern es wird hierdurch eine Verbesserung der Flüssigkeitsaufnahme- und - weiterleitungsfunktion der körperzugewandten Flüssigkeitsaufnahmeschicht erreicht.

Vorzugsweise ist das Flächengewicht des Schichtenverbunds 200 - 500 g/m², insbesondere 300 - 400 g/m². Der gewichtsprozentuale Anteil der körperzugewandten Flüssigkeitsaufnahme- und verteilerschicht zur Gesamtmasse beträgt vorzugsweise 10 - 45%, insbesondere 25 - 30%.

Dabei beträgt die Fläche der Prägepunkte vorteilhafterweise 0,25 bis 4 mm². Der Anteil der Fläche der Prägepunkte zur in Projektion betrachteten Gesamtoberfläche der Flüssigkeitsaufnahmeschicht beträgt vorzugsweise 2 bis 35, insbesondere 5 bis 15 %.

Wie bei der Herstellung der Prägepunkte entsprechend EP 1 032 342 B1 können die Prägepunkte von beiden Seiten vorgesehen sein und einander punktgenau treffen.

Bei einer weiteren bevorzugten Ausführungsform des Schichtenverbunds wird die Prägung auch durch eine auf der körperabgewandten Seite der Saugschicht vorzugsweise vorzusehende Deckschicht hindurch angebracht. Wenn diese Deckschicht in vorteilhafter Weise von einer Tissueschicht gebildet wird, so entsteht eine griffige, im Zuge der Herstellung gut handhabbare Oberflächengestaltung, welche die Speicherschicht vor "Ausfasern" schützt.

Ferner ist Gegenstand der Erfindung ein Hygieneartikel, insbesondere eine Damenbinde oder Slipeinlage, die durch einen Saugkörper der vorstehend beschriebenen erfindungsgemäßen Art gekennzeichnet ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:

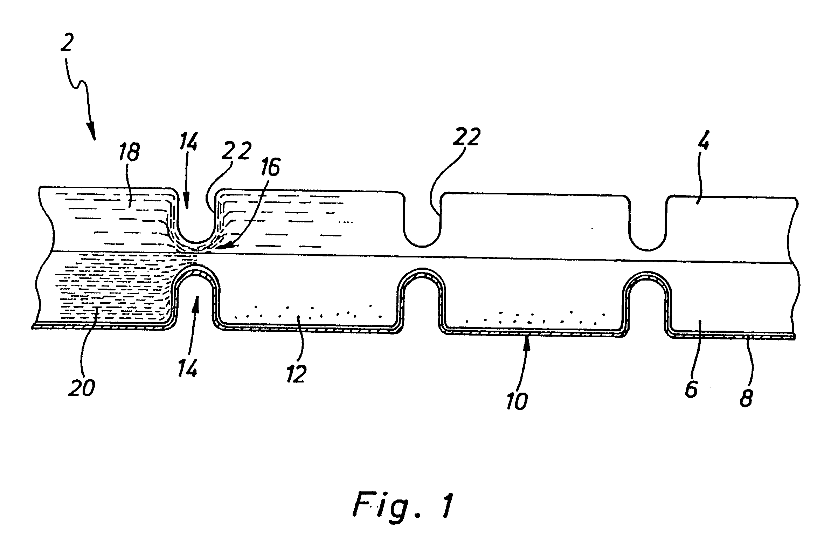

- Figur 1:: eine Querschnittsansicht eines erfindungsgemäßen Saugkörpers für eine Damenbinde.

Die Figur zeigt einen insgesamt mit dem Bezugszeichen 2 bezeichneten Saugkörper für einen Hygieneartikel, beispielsweise eine Damenbinde oder eine Slipeinlage. Der Saugkörper 2 weist eine obere körperzugewandte Flüssigkeitsaufnahme- und -verteilerschicht 4 und eine darunter körperabgewandt angeordnete Flüssigkeitsspeicherschicht 6 sowie eine Tissue-Deckschicht 8 auf der körperabgewandten Seite der Speicherschicht 6 auf.

Die Flüssigkeitsaufnahme- und -verteilerschicht 4 besteht vorzugsweise zu 100 % aus intravernetzten Zellulosefasern, und die Flüssigkeitsspeicherschicht 6 besteht vorzugsweise zu 20 bis 98 Gew.-% aus natürlichen Zellulosefasern (Zellstoff). Sie kann vorzugsweise superabsorbierende Polymermaterialien, sogenannte hydrogelbildende Materialien, umfassen und gegebenenfalls weitere Zusatzstoffe. Vorzugsweise ist die Speicherschicht 6 aber aus natürlichen Zellulosefasern und superabsorbierenden Materialien gebildet.

Auf der körperabgewandten Seite der Speicherschicht 6 ist eine dünne Tissue-Schicht aus Zellulose-Fasern vorgesehen, die sich an die Speicherschicht 6 anschmiegt und eine griffige, verhältnismäßig glatte Oberfläche 10 ausbildet, die ein Ausfasern der Speicherschicht 6 verhindert. Zudem verhindert diese Tissue-Schicht 8, dass partikelförmige superabsorbierende Materialien 12 aus der Speicherschicht austreten.

Bei der Herstellung des dargestellten Schichtenverbunds wird zunächst auf eine in Bahnrichtung geförderte Tissue-Lage eine Mischung aus Zellulosefasern und superabsorbierenden Partikeln im Luftlegeverfahren abgelegt und dann im wesentlichen auf das für eine Speicherschicht geforderte kapillare Porenvolumen zwischen den Zellulosefasern zur Bildung der Speicherschicht 6 vorverdichtet.

Darauf werden intravernetzte Zellulosefasern zur Bildung der Flüssigkeitsaufnahme- und -verteilerschicht 4 abgelegt. Die so gebildete lose Faserschichtung wird einem Kalanderwalzenpaar mit entsprechend EP 1 032 342 B1 vorgesehenen Prägemittel bildenden Noppen zugeführt. Die Noppen sind nach einem regelmäßigen Raster über die Oberfläche der Kalanderwalzen verteilt. Sie haben eine endseitige Prägefläche von 0,2 bis 4 mm².

Die Anordnung der Kalanderwalzen und des Musters der Prägenoppen zueinander sowie die Bestimmung des Kalanderwalzenspaltes ist vorzugsweise derart, dass.sich die endseitigen Prägeflächen der Prägenoppen nahezu berühren, wobei ein von der Dicke der vorverdichteten Faserschichtung und der Masse des Fasermaterials abhängiger Abstand der endseitigen Prägeflächen der Noppen voneinander gewählt wird, und zwar derart, dass eine rasterförmige Verprägung der Schichten gebildet wird, die beim bestimmungsgemäßen Gebrauch, also bei der Handhabung des Verbunds, aber auch in einem Hygieneartikel, sich nicht wieder löst.

Figur 1 zeigt in der Schnittansicht den wie vorbestehend beschrieben kalandrierten Schichtenverbund. Man erkennt Prägestellen 14, die sich von beiden Seiten des Saugkörpers 2 im wesentlichen senkrecht zur Bahnebene in das Saugkörpermaterial hineinerstrecken. Es verbleibt ein in der Figur nicht maßstabsgetreu dargestellter hochverdichteter Bereich 16, in dem die Fasern der Schichten 4, 6, aber auch 8, unlösbar miteinander verpresst sind und so einen unlösbaren Schichtenverbund bilden.

Wie in Figur 1 links angedeutet, ist das Porenvolumen, also der Faserzwischenraum 18 der die Schicht 4 bildenden Fasern sehr viel größer als derjenige 20 der Verteilerschicht, die demzufolge eine sehr hohe Kapillarität aufweist, was mit der Fähigkeit einhergeht, Flüssigkeit an von der Auftreffstelle entfernte noch ungenutzte Bereiche der Speicherschicht 6 zu transportieren.

Es hat sich nun herausgestellt, dass die Herstellung von rasterförmigen Prägepunkten 14 in der Flüssigkeitsaufnahmeschicht 4 in vorteilhafter Weise zur Ausbildung von trichterförmig verlaufenden Porengradienten in der Flüssigkeitsaufnahmeschicht 4 führt. Hierdurch wird eine Vielzahl von lokalen Drainagestellen innerhalb der Flüssigkeitsaufnahmeschicht 4 gebildet, die im übrigen ein größeres Porenvolumen als die darunter liegende Speicherschicht 6 aufweist. Es wird also an dem Konzept der großporigen Flüssigkeitsaufnahmeschicht festgehalten, wobei punktuell und rasterförmig durch die punktförmige Verprägung der Schichten Drainagestellen innerhalb dieser Aufnahmeschicht gebildet werden, die - wie sich zeigte - die Einleitung von Flüssigkeit in die darunter liegende Speicherschicht begünstigen. Ferner stellen die Prägestellen 14 ein weiteres der ersten Aufnahme von auftreffender Flüssigkeit dienendes Aufnahmevolumen 22 dar.

Der vorstehend beschriebene Saugkörper 2 kann als Saugkörper in einem Hygieneartikel Verwendung finden, wobei auf der körperabgewandten Seite vorzugsweise eine flüssigkeitsundurchlässige Folienschicht vorgesehen wird und auf der körperzugewandten Seite könnte eine flüssigkeitsdurchlässige Vliesstoffabdeckung als Topsheet-Material vorgesehen sein. Gegebenenfalls können zwischen Topsheet und Saugkörper eine oder mehrere weitere als Verteilerschichten dienende großporige Materialien, vorzugsweise Vliesstoffe, insbesondere auf Polyesterbasis, angeordnet sein.

## Patentansprüche

1. Saugkörper (2) für Hygieneartikel, insbesondere Damenbinde oder Slipeinlage, mit einer körperzugewandten Flüssigkeitsaüfnahme- und verteilerschicht (4) aus einem ersten Fasermaterial mit einer ersten Porengröße und einer körperabgewandten Flüssigkeitsspeicherschicht (6) mit wenigstens 20-98 Gew.-% Zellstofffasern mit einer zweiten Porengröße, die gegenüber der ersten geringer ist, wobei das erste Fasermaterial aus luftgelegten intravernetzten Cellulosefasern besteht und wobei die Schichten (4, 6) durch eine Vielzahl von rasterartig vorgesehenen Prägepunkten (14) klebstofffrei miteinander verbunden sind, und wobei dadurch in der Flüssigkeitsaufnahme und -verteilerschicht (4) um die Prägepunkte (14) herum ein trichterförmiger Porengradient in dieser Schicht ausgebildet ist, der die Flüssigkeitseinleitung in die darunter liegende Speicherschicht (6) unterstützt.

2. Saugkörper nach Anspruch 1, **dadurch gekennzeichnet, dass** das Flächengewicht des Schichtenverbunds 200 - 500 g/m², insbesondere 300 - 400 g/m² beträgt.

3. Saugkörper nach Anspruch 1 oder 2, **dadurch gekennzeichnet, dass** der gewichtsprozentuale Anteil der körperzugewandten Flüssigkeitsaufnahme- und verteilerschicht (4) zur Gesamtmasse 10 - 45 %, insbesondere 25 - 30 % beträgt.

4. Saugkörper nach Anspruch 1, 2 oder 3, **dadurch gekennzeichnet, dass** die Fläche der Prägepunkte (14) 0,25 bis 4 mm² beträgt.

5. Saugkörper nach einem der vorstehenden Ansprüche,
**dadurch gekennzeichnet, dass** die Prägepunkte (14) von beiden Seiten des Schichtenverbunds vorgesehen sind.

6. Saugkörper nach Anspruch 5, **dadurch gekennzeichnet, dass** die Prägepunkte (14) von beiden Seiten des Schichtenverbünds vorgesehen sind und einander punktgenau treffen.

7. Saugkörper nach einem der vorstehenden Ansprüche, **dadurch gekennzeichnet, dass** auf der körperabgewandten Seite der Saugschicht (6) eine Deckschicht (8) vorgesehen ist, die von den Prägepunkten miterfasst ist.

8. Saugkörper nach einem der vorstehenden Ansprüche, **dadurch gekennzeichnet, dass** die Deckschicht (8) eine Tissue-Schicht ist.

9. Hygieneartikel, insbesondere Damenbinde oder Slipeinlage, **gekennzeichnet durch** einen Saugkörper (2) nach einem oder mehreren der vorstehenden Ansprüche.

## Claims

1. Absorbent body (2) for hygiene articles, particularly sanitary napkins or panty liners, having a fluid acquisition and distribution layer (4) facing the body made of a first fibrous material with a first pore size and a fluid storage layer (6) facing away from the body with at least 20 - 98% by weight of cellulose fibers with a second pore size, which is smaller compared with the first, wherein the first fibrous material consists of air-laid, intracrosslinked cellulose fibers and wherein the layers (4, 6) are bonded without adhesive by a plurality of embossing points (14) arranged in a grid pattern and wherein, as a result, around the embossing points (14) in the fluid acquisition and distribution layer (4) a funnel-shaped pore gradient is formed in this layer which promotes the introduction of fluid into the storage layer (6) disposed thereunder.

2. Absorbent body in accordance with claim 1, **characterized in that** the base weight of the composite layer measures 200 - 500 g/m², especially 300 - 400 g/m².

3. Absorbent body in accordance with claim 1 or 2, **characterized in that** the weight by percent of the fluid acquisition and distribution layer 4 is 10 - 45 %, especially 25 - 30 % of the total mass.

4. Absorbent body in accordance with claims 1, 2 or 3, **characterized in that** the area of the embossing points (1) measures 0,25 to 4 mm².

5. Absorbent body in accordance with one of the preceding claims, **characterized in that** the embossing points (14) are provided from both sides of the composite layer.

6. Absorbent body in accordance with claim 5, **characterized in that** the embossing points (14) are furnished from both sides of the composite layer and align w ith each other exactly.

7. Absorbent body in accordance with one of the preceding claims, **characterized in that** a top sheet (8) is furnished on the side of the absorbent layer (6) facing away from the body, said sheet also incorporating the embossing points.

8. Absorbent body in accordance with one of the preceding claims, **characterized in that** the top sheet (8) is a tissue sheet.

9. Hygiene article, particularly sanitary napkin or panty liner, **characterized by** an absorbent body (2) in accordance with one or more of the preceding claims.

## Revendications

1. Corps absorbant (2) pour articles d'hygiène, en particulier serviette hygiénique ou protège-slip, comprenant une couche d'absorption et de répartition de liquide (4) orientée vers le corps, réalisée dans une première matière fibreuse ayant une première grosseur de pores, et une couche d'accumulation de liquide (6) opposée au corps ayant au moins 20 à 98 % en poids de fibres de cellulose, avec une deuxième grosseur de pores qui est plus réduite que la première, la première matière fibreuse étant constituée de fibres de cellulose intraréticulées par voie sèche, les couches (4, 6) étant reliées les unes aux autres sans colle par une multitude de points de gaufrage (14) prévus à la manière d'un réseau et, dans la couche d'absorption et de répartition de liquide (4), un gradient de pore en forme de cône étant ainsi formé dans cette couche autour des points de gaufrage (14), ledit gradient aidant à la répartition de liquide dans la couche d'accumulation (6) située au-dessous.

2. Corps absorbant selon la revendication 1, **caractérisé en ce que** le poids par surface du composé de couches est compris entre 200 et 500 g/m², en particulier entre 300 et 400 g/m².

3. Corps absorbant selon la revendication 1 ou 2, **caractérisé en ce que** la part de pourcentage en poids de la couche d'absorption et de répartition de liquide (4) orientée vers le corps par rapport à la masse totale représente 10 à 45%, en particulier 25 à 30%.

4. Corps absorbant selon la revendication 1, 2 ou 3, **caractérisé en ce que** la surface des points de gaufrage (14) représente 0,25 à 4 mm².

5. Corps absorbant selon l'une des revendications précédentes, **caractérisé en ce que** les points de gaufrage (14) sont prévus des deux côtés du composé de couches.

6. Corps absorbant selon la revendication 5, **caractérisé en ce que** les points de gaufrage (14) sont prévus des deux côtés du composé de couches et coïncident exactement entre eux.

7. Corps absorbant selon l'une des revendications précédentes, **caractérisé en ce qu'**une couche de recouvrement (8), qui est incluse par les points de gaufrage, est prévue du côté de la couche absorbante (6) opposée au corps.

8. Corps absorbant selon l'une des revendications précédentes, **caractérisé en ce que** la couche de recouvrement (8) est une couche de papier crêpé.

9. Article d'hygiène, en particulier serviette hygiénique ou protège-slip, **caractérisé par** un corps absorbant (2) selon l'une ou plusieurs des revendications précédentes.
